# EUROPÄISCHE PATENTSCHRIFT

(11) **EP 1 694 291 B1**
(45) Veröffentlichungstag und Bekanntmachung des Hinweises auf die Patenterteilung: **11.11.2009**
(21) Anmeldenummer: 04798121.2
(22) Anmeldetag: 30.11.2004
(51) Int. Cl.: A61K 9/00, A61K 31/495, A61K 38/15

(54) **ENDOPARASITIZIDE MITTEL ZUR TOPISCHEN APPLIKATION**
ENDOPARASITICIDAL AGENTS FOR TOPICAL APPLICATION
AGENTS ENDOPARASITICIDES A APPLICATION TOPIQUE

(30) Priorität: 13.12.2003 DE 10358525
(43) Veröffentlichungstag der Anmeldung: 30.08.2006
(73) Patentinhaber: Bayer Animal Health GmbH, 51368 Leverkusen (DE)
(72) Erfinder: KANIKANTI, Venkata-Rangarao, 51381 Leverkusen (DE); BACH, Thomas, 42349 Wuppertal (DE); ALTREUTHER, Gertraut, 42105 Wuppertal (DE); TRÄUBEL, Michael, 50733 Köln (DE); HAMANN, Hans-Jürgen, 41539 Dormagen (DE)
(86) Internationale Anmeldenummer: PCT/EP2004/013552
(87) Internationale Veröffentlichungsnummer: WO 2005/055973

(56) Entgegenhaltungen:
- DE-A1- 3 619 030
- DE-A1- 4 400 464
- DE-A1- 19 921 887
- US-A- 4 988 696
- US-B1- 6 340 672
- MAGNUSSON B M ET AL: "Veterinary drug delivery: potential for skin penetration enhancement." ADVANCED DRUG DELIVERY REVIEWS. 1 SEP 2001, Bd. 50, Nr. 3, 1. September 2001 (2001-09-01), Seiten 205-227, XP002329632 ISSN: 0169-409X

## Beschreibung

Die vorliegende Erfindung betrifft transdermal applizierbare Mittel, enthaltend cyclische Depsipeptide und/oder Praziquantel, sowie ihre Herstellung und Verwendung zur Bekämpfung von Endoparasiten.

Der anthelmintische Wirkstoff Praziquantel (US P 4 001 411) wird üblicherweise oral verabreicht, siehe z. B. DE-A-199 41 024, WO 98/03157, US 2002/0081292 A1 und WO 97/25976. Bei topischer Applikation von Endoparasitiziden muß der Wirkstoff durch die Haut in den Blutkreislauf gelangen, um die betreffenden Endoparasiten zu erreichen. Da Praziquantel sich wenig zur transdermalen Applikation eignet, ist bei diesem Wirkstoff die topische transdermale Applikationsform aufgrund der zu erwartenden Schwierigkeiten unüblich, insbesondere bei Hunden. Ein Mittel zur dermalen Behandlung von Wurmerkrankungen mit Praziquantel wird in EP-A-267 404 beschrieben. Die Anwendung dieses Mittels ist jedoch auf die Katze beschränkt, bei der wirksame transdermale Applikation in der Regel wesentlich leichter zu erreichen ist als z. B. bei Hunden.

WO 01/60380 (Phoenix Scientific, Inc.) offenbart parasitizide Formulierungen zur Injektion oder zur Pour-on-Anwendung, die ein Pyrrolidon-Lösungsmittel, ein weiteres Lösungsmittel und einen parasitiziden Wirkstoff enthalten können. In der umfangreichen Liste der Wirkstoffe wird unter anderem auch Praziquantel genannt.

EP-A-1 308 163 (Wyeth) offenbart endoparasitizide Mittel in Gelform, die Moxidectin, Praziquantel, Benzylalkohol, Ethanol, kolloidales Siliziumdioxid, ein Tensid und ein Öl enthalten.

WO 95/23590 (Bomac Laboratories) offenbart ein kompliziertes Verfahren zur Herstellung von anthelmintischen Mitteln zur dermalen Applikation. Die Mittel enthalten einen Träger, einen Emulgator, ein Öl und ein Verdünnungsmittel. Als Wirkstoffe kommen vor allem Benzimidazole in Frage, unter anderem werden jedoch auch makrocyclische Lactone und Praziquantel genannt.

WO 02/094288 beschreibt ein Arzneimittel für Tiere, das ein Avermectin-Oxim-Derivat, insbesondere Selamectin, in Kombination mit Praziquantel enthält. Es wird auch die topische Applikation vorgeschlagen, entsprechende Formulierungen enthalten einen Di(C₂₋₄-glykol)mono(C₁₋₄-alkyl)ether und ein optionales hautverträgliches Lösungsmittel.

Ein cyclisches Depsipeptid PF1022 und seine Wirkung gegen Endoparasiten ist bekannt aus EP-A 382 173.

Weitere cyclische Depsipeptide und ihre endoparasitizide Wirkung sind Gegenstand der deutschen Patentanmeldungen EP-A 626 376; EP-A 626 375; EP-A 644 883.

Endoparasitizide Mittel enthaltend Praziquantel oder Epsiprantel und cyclische Depsipeptide sind in

EP 662 326 beschrieben.

Gegenstand der WO 96/38165 sind endoparasitizide Mittel enthaltend Avermectine, Ivermectine, Milbemycine in Kombination mit cyclischen Depsipeptiden sowie gegebenenfalls Praziquantel oder

Epsiprantel.

Transdermal applizierbare Mittel enthaltend cyclische Depsipeptide zur Bekämpfung von Endoparasiten sind in WO 01/62268 beschrieben.

DE 199 21 887 A1 offenbart Mittel mit endoparasitizider Wirkung, enthaltend ein cyclisches Depsipeptid (Emodepside), N-Methylpyrrolidon als Lösungsmittel, und resorptionsfördernde Stoffe, z.B. Fettalkohole.

Sogenannte Permeations- oder Penetrationsenhancer, welche die transdermale Applikation von Arzneimitteln verbessern, sind im Prinzip im Stand der Technik bekannt, siehe z. B. Sinha et al. in Drug Development and Industrial Pharmacy, 26(11), 1131-1140 (2000); Clarys et al. European Jounal of Pharmaceutics and Biopharmaceutics 46 (1998), 279-283 und im Kapitel 6 von Dermatopharmazie (Wissenschaftliche Verlagsgesellschaft mbH Stuttgart 2001).

Die Wirkungshöhe und/oder Wirkungsdauer der vorbekannten Mittel ist jedoch, insbesondere bei bestimmten Wirtstieren, gegen bestimmte Organismen und/oder bei niedrigen Anwendungskonzentrationen, nicht in allen Anwendungsgebieten völlig zufriedenstellend.

Wegen der vielfältigen Anforderungen an moderne Arzneimittel, beispielsweise was Wirkhöhe (z. B. Plasmakonzentration des Wirkstoffs), Wirkdauer, Wirkspektrum, Anwendungsspektrum, Toxizität, Kombination von Wirkstoffen, Kombination mit Formulierungshilfsmitteln und wegen des möglichen Auftretens von Resistenzen kann die Entwicklung neuer Arzneimittel nie als abgeschlossen betrachtet werden, und es besteht beständig ein hoher Bedarf an neuen Mitteln, die zumindest in Teilaspekten Vorteile gegenüber den bekannten Mitteln bringen.

Um dem Tierhalter eine möglichst einfache Applikation endoparasitizider Wirkstoffe zü ermöglichen, ist es wünschenswert ein transdermal applizierbares Mittel zu Verfügung zu stellen

Wie aus der Literatur bekannt ist, sind Moleküle mit Molekulargewichten >1000 u äußerst schlecht bei topischer Applikation durch die Haut zu penetrieren. Besonders schlecht penetrieren Peptide oder Proteine mit größeren Molekulargewichten (Cevc et al, Advanced Drug Delivery Reviews 18 (1996) 349-378; Bauer et al. Pharmazeutische Technologie, 1993, S. 364, Thieme Verlag; Gurny et al. Dermal and Transdermal Drug Delivery, 1993, S. 131, Wissenschaftliche Verlagsgesellschaft). Die Penetration ist jedoch bei endoparasitiziden Wirkstoffen Voraussetzung, da sie gegen Endoparasiten z. B. im Magen-Darm-Trakt zur Wirkung kommen sollen.

Obwohl im Stand der Technik vereinzelt vorgeschlagen wurde, Praziquantel bzw. cyclische Depsipeptide topisch zu applizieren, ist es dem Fachmann bekannt, dass diese Wirkstoffe hierfür sehr wenig geeignet sind und die bekannten Formulierungen daher nicht völlig befriedigend sind, insbesondere beispielsweise bei den sogenannten dosistreibenden Würmern wie dem Peitschenwurm *Trichuris vulpis* und/oder dem Bandwurm *Taenia canis.*

Aufgabe der vorliegenden Erfindung ist daher ein topisch applizierbares endoparasitizides Mittel bereitzustellen, das folgende Eigenschaften aufweist:
- Gute transdermale Wirkung bei verschiedenen Wirtstieren, insbesondere Hunden, auch bei dosistreibenden Würmern (z. B. *Trichuris vulpis, Taenia canis)*
- Gute Hautverträglichkeit
- Langzeitstabilität
- Anwenderfreundlichkeit

Gegenstand der vorliegenden Erfindung sind:
Mittel enthaltend:
   - als Wirkstoff ein cyclisches Depsipeptid und/oder Praziquantel
   - ein Pyrrolidon-Lösungsmittel
   - einen Terpen-Penetrationsenhancer.

Gegenstand der vorliegenden Erfindung ist ferner die Herstellung der vorstehend genannten Mittel sowie ihre Verwendung zur Bekämpfung von Endoparasiten.

Depsipeptide sind den Peptiden ähnlich und unterscheiden sich von diesen darin, daß ein oder mehrere α-Aminosäurebausteine durch α-Hydroxycarbonsäurebausteine ersetzt sind. Bevorzugte cyclische Depsipeptide sind solche mit 18 bis 24 Ringatomen, insbesondere mit 24 Ringatomen.

Zu den Depsipeptiden mit 18 Ringatomen zählen Verbindungen der allgemeinen Formel (I): 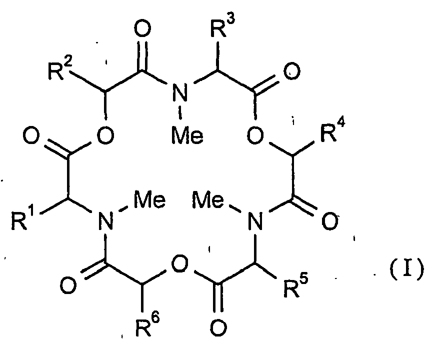 in welcher
- R¹, R³ und R⁵: unabhängig voneinander für Wasserstoff geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylammnoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,
- R², R⁴ und R⁶: unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl gegebenenfalls substituiertes Aryl oder Arylalkyl wobei als Substituenten genannt seien Halogen, Hydroxy, Alkyl, Alkoxy, stehen,
sowie deren optische Isomere und Racemate.

Bevorzugt sind Verbindungen der Formel (I), 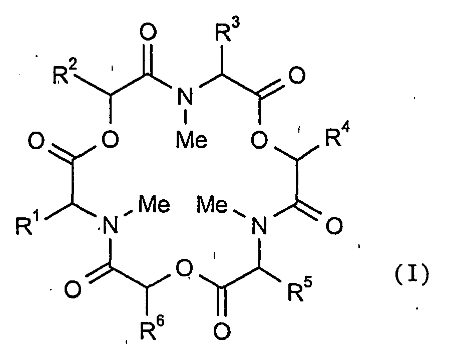 in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-hydroxymethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methyl aminobutyl, C₁-C₄-Dialkylarnino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl-(Fmoc)amino-C₁-C₆-alkyl, insbesondere 9-Fluorenyl-methoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl; insbesondere Mercaptomethyl, C₁-C₄-Alkyylthio-C₁-C₆-alkyl, insbesondere Methylthioethyl, C₁-C₄-Alkylsulfmyl-C₁-C₆-alkyl, insbesondere Methylsulfinylethyl, C₁-C₄-A1kylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cyaoalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann,
sowie deren optische Isomere und Racemate.

Besonders bevorzugt sind Verbindungen der Formel ( I ),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch einen oder mehrere gleiche oder verschiedene der oben angegebenen Reste substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, Aryl-C₁-C₄-alkyloxy-C₁-C₆-alkyl, insbesondere Benzyloxymethyl, 1-Benzyloxyethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonylmethyl, C₁-C₄-Alkylanüno-C₁-C₆-alkyl, insbesondere Methylaminopropyl, Methylaminobutyl, C₁-C₄-Dialkylanuno-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch einen oder mehrere gleiche oder verschiedene der oben angegebenen Reste substituiert sein kann,
sowie deren optische Isomere und Racemate.

Ganz besonders bevorzugt sind Verbindungen der Formel (I),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl C₂-C₈-Alkenyl, insbesondere Allyl, C₃-C₇-Cyaoalkyl-C₁-C₄-alkyl, insbesondere Cyclohexylmethyl, Phenyl-C₁-C₄-alkyl, insbesondere Phenylmethyl,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈- Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl das gegebenenfalls durch einen oder mehrere gleiche oder verschiedene der oben angegebenen Reste substituiert sein kann,
sowie deren optische Isomere und Racemate.

Im Sinne der vorliegenden Erfindung können alle Verbindungen der allgemeinen Formel (I), die in optisch aktiven, stereoisomeren Formen oder als racemische Gemische vorliegen können, verwendet werden. Vorzugsweise werden jedoch die optisch aktiven, stereoisomeren Formen der Verbindungen der allgemeinen Formel (I) erfindungsgemäß verwendet.

Im einzelnen seien folgende Verbindungen der allgemeinen Formel (I) genannt, in welcher die Reste R¹ bis R⁶ die folgende Bedeutung haben:

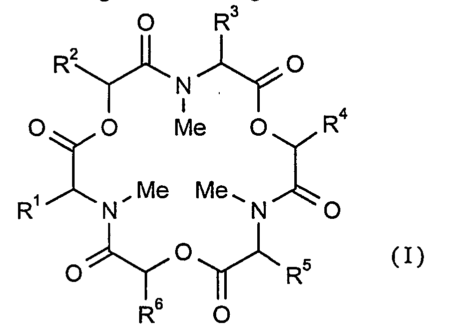

| **R¹** | **R²** | **R³** | **R⁴** | **R⁵** | **R⁶** |
|---|---|---|---|---|---|
| -CEMeCH₂Me | -Cyclohexyl | -CHMeCH₂Me | -Me | -CHMeCH₂Me | -Me |
| -CHMeCH₂Me | -Cyclohexyl | -CHMeCH₂Me | -Me | -CHMeCH₂Me | -Cyclohexyl |
| -CHMeCH₂Me | -CH₂-Phe | -CHMeCH₂Me | -Me | -CHMeCH₂Me | -Me |
| -CHMeCH₂Me | -CH₂-Phe | -CHMeCH₂Me | -Me | -CHMeCH₂Me | -CH₂-Phe |
| -CHMeCH₂Me | -(CH₂)₃-Me | -CHMeCH₂Me | -Me | -CHMeCH₂Me | -Me |
| -CHMeCH₂Me | -(CH₂)₃-Me | -CHMeCH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CH₂-CH=CH₂ | -Me | -CH₂-CH=CH₂ | -Me | -(CH₂)-CH=CH₂ | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -cyclohexyl | -Me | -Cyclohexyl | -Me | -Cyclohexyl | -Me |
| -CH₂CHMe₂ | -Cyclohexyl | -CH₂CHMe₂ | -Me | -CH₂CHMe₂ | -Cyclohexyl |
| -CH₂CHMe₂ | -Cyclohexyl | -CH₂CHMe₂ | -Me | -CH₂CHMe₂ | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -Cyclohexyl | -Me | -Cyclohexyl | -Me | -Cyclohexyl | -Me |
| -CHMe₂ | -CHMe₂ | -CHMe | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CHMe₂ | -CHMe₂ | -CH₂-Me | -Me |
| -(CH₂)₂-Me | -CHMe₂ | -(CH₂)₂-Me | -Me | -(CH₂)₂-Me | -Me |
| -(CH₂)₂-Me | -CHMe₂ | -(CH₂)₂-Me | -CHMe₂ | -(CH₂)₂-Me | -Me |
| -(CH₂)₃-Me | -CHMe₂ | -(CH2)₃-Me | -Me | -(CH₂)₃-Me | -Me |
| -(CH₂)₃-Me | -CHMe₂ | -(CH₂)₃-Me | -CHMe₂ | -(CH₂)₃-Me | -Me |
| -CH₂-CH=CH₂ | -CHMe₂ | -CH₂CH=CH₂ | -Me | -CH₂-CH=CH₂ | -Me |
| -CH₂-CH=CH₂ | -CHMe₂ | -CH₂CH=CH₂ | -CHMe₂ | -CH₂-CH=CH₂ | -Me |
| -Me | -Me | -CHMeCH₂Me | -Me | -CH₂-Me | -Me |
| -Me | -Me | -CHMeCH₂Me | -Me | -(CH₂)₃-Me | -Me |

| | | | | | |
|---|---|---|---|---|---|
| Me = Methyl; Phe = Phenyl | | | | | |

Weiterhin sei als Depsipeptid die aus EP-OS 382 173 bekannte Verbindung PF 1022 der folgenden Formel (IIa) genannt:

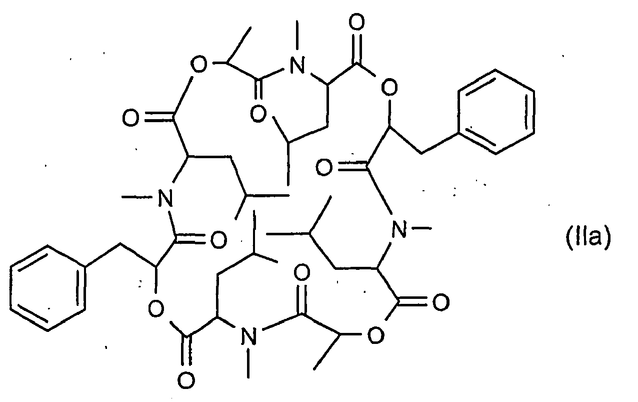

Außerdem seien als Depsipeptide die aus der PCT-Anmeldung WO 93/19053 bekannten Verbindungen genannt

Insbesondere seien aus WO 93/19053 die Verbindungen der folgenden Formel (IIb) genannt: 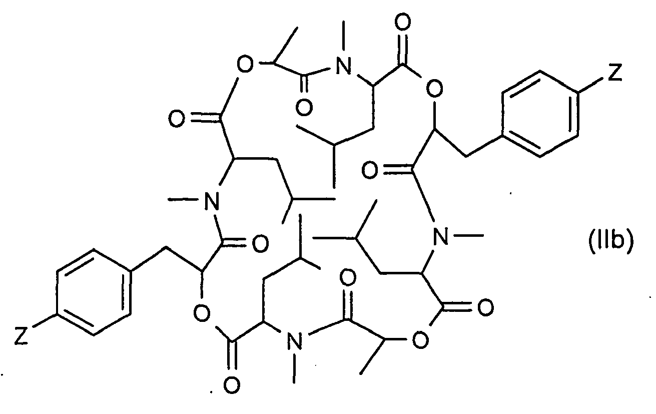 in welcher
- Z: für N-Morpholinyl, Amino, Mono- oder Dimethylamino steht.

Außerdem seien Verbindungen der folgenden Formel (IIc) genannt: 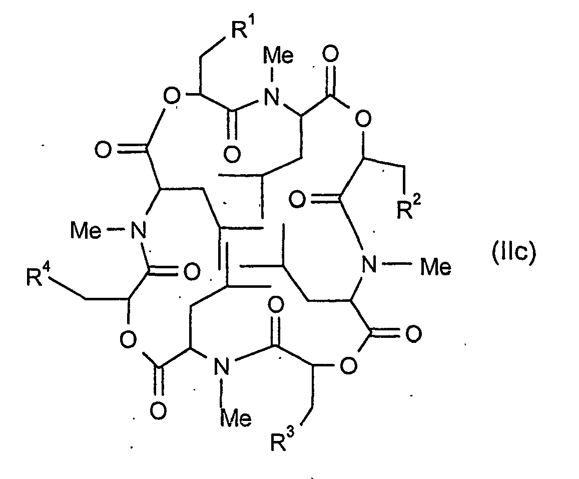 in welcher
- R¹, R², R³, R⁴: unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formel (I) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP=A-669 343, EP-A-787 141, EP-A-865 498, EP-A-903 347 beschriebenen Verfahren erhalten werden.

Zu den cyclischen Depsipeptiden mit 24 Ringatomen zählen auch Verbindungen der allgemeinen Formel (IId) 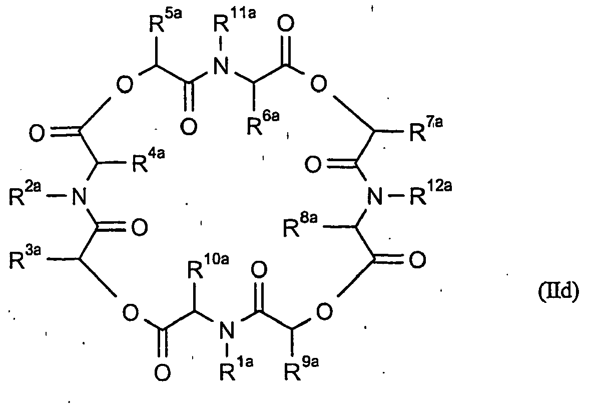 in welcher
- R^{1a}, R^{2a}, R^{11a} und R^{12a}: unabhängig voneinander für C₁₋₈-Alkyl, C₁₋₈-Halogenalkyl, C₃₋₆-Cycloalkyl, aralkyl, Aryl stehen,
- R^{3a}, R^{5a}, R^{7a}, R^{9a}: unabhängig voneinander für Wasserstoff oder geradkettiges oder verzweigtes C₁₋₈-Alkyl steht, das gegebenenfalls durch Hydroxy, C₁₋₄-Alkoxy, Carboxy, 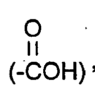 Carboxamid, 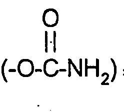 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
- R^{4a}, R^{6a}, R^{8a}, R^{10a}: unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen
sowie deren optische Isomere und Racemate.

Bevorzugt werden Verbindungen der Formel (IId) eingesetzt, in welcher
- R^{1a}, R^{2a}, R^{11a} und R^{12a}: unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
- R^{3a} bis R^{10a}: die oben angegebene Bedeutung haben.

Besonders bevorzugt sind Verbindungen der Formel (IId), in welcher
- R^{1a}, R^{2a}, R^{11a} und R^{12a}: unabhängig voneinander für Methyl, Ethyl, Propyl, Isopropyl oder n-, s-, t-Butyl stehen,
- R^{3a}, R^{5a}, R^{7a}, R^{9a}: für Wasserstoff, geradkettiges oder verzweigtes C₁₋₈-Akyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
- R^{4a}, R^{6a}, R^{8a}, R^{10a}: unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die Verbindungen der Formel (IId) können ebenfalls nach den in EP-A-382 173, DE-A 4 317,432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787.141, EP-A-865 498, EP-A-903 347 beschriebenen Verfahren erhalten werden.

Erfindungsgemäß ganz besonders bevorzugte Depsipeptide sind PF 1022 A (siehe Formel (IIa) und Emodepside (PF 1022-221, Verbindung der Formel (IIb) worin beide Reste Z für den Morpholinylrest stehen). Der INN Emodepside steht für die Verbindung mit dem systematischen Namen: Cyclo[(*R*)-lactoyl-*N*-methyl-L-leucyl-(*R*)-3-(p-morpholinophenyl)lactoyl-*N*-methyl-L-leucyl-(*R*)-lactoyl-*N*-metlmyl-L-leucyl-(*R*)-3-(*p*-morpholinophenyl)lactoyl-*N*-metlmyl-L-leucyl.

Praziquantel ist als Wirkstoff gegen Endoparasiten lange bekannt (vgl. z. B US 4 001 411), entsprechende Produkte sind z. B. unter der Bezeichnung Droncit® im Handel erhältlich.

Die erfindungsgemäßen Mittel können entweder ein oder mehrere der vorstehend genannten cyclischen Depsipeptide oder Praziquantel als Wirkstoff enthalten. Bevorzugt ist jedoch die Kombination, d.h. ein Mittel, das sowohl ein cyclisches Depsipeptid als auch Praziquantel enthält, und zwar insbesondere Emodepside und Praziquantel.

Die besonderen Vorteile der erfindungsgemäßen Mittel sind zu einem erheblichen Teil auf die hierfür speziell ausgewählten Penetrationsenhancer zurückzuführen. Penetrationsenhancer sind Verbindungen, welche die topische transdermale Applikation pharmazeutischer Wirkstoffe verbessern. In der Literatur sind zahlreiche verschiedene Verbindungen und Verbindungsklassen als Penetrationsenhancer beschrieben. Es zeigt sich jedoch, dass diese in Abhängigkeit von den eingesetzten Wirkstoffen und sonstigen Hilfsstoffen zu sehr unterschiedlichen Ergebnissen führen. Zum Teil ist der Transport durch die Haut nicht ausreichend oder es gibt Probleme mit der Hautverträglichkeit.

Bei den. Versuchen mit Praziquantel und/oder den cyclischen Depsipeptiden zeigte sich, dass mit zahlreichen in der Literatur beschriebenen Penetrationsenhancem keine für die praktische Anwendung geeigneten Ergebnisse erzielt werden können, wohingegen die erfindungsgemäßen Mittel, vor allem wegen der speziell ausgewählten Penetrationsenhancer, ausgezeichnete Ergebnisse liefern.

Terpen-Penetrationsenhancer sind beschrieben in Kapitel 6 von "Dermatopharmazie" (Wissenschaftliche Verlagsgesellschaft mbH Stuttgart 2001). Typische Gruppen von Terpen-Penetrationsenhancern sind die Terpen-Kohlenwasserstoffe, wie z. B. Limonen, α- Pinen oder β-Caren; Terpenalkohole, wie z. B. α-Terpineol, Terpinen-4-ol oder Carveol; Terpen-Ketone, wie z. B. Carvon, Pulegon, Piperiton oder Menthon und die Terpen-Oxide, wie z. B. Limonenoxid, α-Pinenoxid, 1,8-Cineol und, die verwandten Verbindungen Cyclohexenoxid oder Cyclopentenoxid. Von diesen bevorzugt sind die Terpenkohlenwasserstoffe, inbesondere Limonen. Die verwendbaren Terpen-Penetrationsenhancer haben in der Regel ein Grundgerüst mit 10 Kohlenstoffatomen.

Der Terpen-Penetrationsenhancer wird üblicherweise in Mengen bis 25 Gew.-% eingesetzt, bevorzugt 2 bis 20 Gew.-%, besonders bevorzugt 5 bis 15 Gew.%.

Aliphatische Fettsäuren sind z. B. beschrieben in Kapitel 6 von "Dermatopharmazie" (Wissenschaftliche Verlagsgesellschaft mbH Stuttgart 2001). Üblicherweise haben diese 5 bis 18 Kohlenstoffatome. Weiterhin sind insbesondere flüssige Fettsäuren vorteilhaft, beispielsweise solche mit ein oder zwei Doppelbindungen. Als gesättigte Fettsäuren seien beispielsweise genannt Valerian-, Capron-, Capryl-, Caprin- Laurin-, Myristin-, Stearin-, Pelargon-, Isovalerian-, Neopentan-, Neoheptan-, Neononan-, Neodecan und Isostearinsäure. Als ungesättigte Fettsäuren seien genannt Ölsäure, Linolsäure und Linolensäure. Besonders bevorzugt sind Linolsäure und insbesondere Ölsäure.

Aliphatische Fettalkohole in Frage, auch diese sind z. B. beschrieben in Kapitel 6 von "Dennatopharmazie" (Wissenschaftliche Verlagsgesellschaft mbH Stuttgart 2001). Geeignete aliphatische Fettalkohole haben vorzugsweise 8 bis 18 Kohlenstoffatome. Als Beispiele seien Capryl-, Decyl-, Lauryl-, 2-Lauryl-, Myristyl-, Cetyl-, Stearyl-, Isostearyl-, Oleoyl-, Linolyl- und Linolenylalkohol genannt. Besonders bevorzugt ist Isostearylalkohol.

Die aliphatischen Fettsäuren und die aliphatischen Fettalkohole werden üblicherweise in Mengen von bis zu 25 Gew.-% eingesetzt, bevorzugt 1 bis 20 %, besonders bevorzugt 2 bis 15 %.

Es ist möglich als Penetrationsenhancer in den erfindungsgemäßen Mitteln einen Terpen-Penetrationsenhancer und eine als Penetrationsenhancer wirkende aliphatische Fettsäure oder einen als Penetrationsenhancer wirkende aliphatischen Fettalkohol einzusetzen. Es können such Kombinationen dieser Penetrationsenhancer-Typen verwendet werden.

Bevorzugt ist der kombinierte Einsatz eines Terpen-Penetrationsenhancers mit einer aliphatischen Fettsäure oder einem aliphatischen Fettalkohol, wobei eine überraschende synergistische Wirkungssteigerung auftritt. Ganz besonders bevorzugt ist die Kombination von Limonen und Ölsäure.

Soweit nicht anders angegeben handelt es sich bei den Prozent-Angaben zur Menge der einzelnen Bestandteile der erfindungsgemäßen Mittel um Gewichtsprozent bezogen auf das Gesamtgewicht des fertigen Mittels.

Die Wirkstoffe sollten in den erfindungsgemäßen Mitteln in relativ hohen Konzentrationen vorliegen, um einerseits die transdermale Wirkung zu verbessern - z. B. bei dosistreibenden Würmern wie *Trichuris vulpis -* und um andererseits das zu applizierende Volumen klein zu halten. Allerdings besteht bei solchen hoch konzentrierten Mitteln bei topischer Applikation (z. B. spot-on) die Gefähr, dass der Wirkstoff auf dem Fell oder der Haut kristallisiert, was generell unerwünscht ist und in der Regel die Hautpenetration verschlechtert. Überraschenderweise wurde gefunden, dass der Zusatz aliphatischer Fettsäuren die Kristallisation der Wirkstoffe verhindert und die Formulierung daher über einen langem Zeitraum für die transdermale Absorption zur Verfügung steht.

Die erfmdungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhältung möglich ist Zu den pathogenen Endoparasiten zählen Cestoden, Trematoden, Nematoden, Acantocephalen:

Mit Praziquantel lassen sich vor allem folgende Endoparasiten bekämpfen:
Aus der Ordnung der Pseudophyllidea z.B.: Diphyllobothrium spp., Spirometra spp., Schistocephalus spp., Ligula spp., Bothridium spp., Diphlogonoporus spp..
Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..
Aus der Unterklasse der Monogenea z.B.: Gyrodactylus spp., Dactylogyrus spp., Polystoma spp..
Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Mit cyclischen Depsipeptiden lassen sich vor allem folgende Endoparasiten bekämpfen:
Aus der Ordnung der Enoplida z.B.: Trichuris spp., Capillaria spp., Trichomosoides spp., Trichinella spp..
Aus der Ordnung der Rhabditia z.B.: Micronema spp., Strongyloides spp..
Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,
Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.,
Aus der Ordnung der Oxyurida z.B.: Oxyuris spp., Enterobius spp., Passalurus spp., Syphacia spp., Aspiculuris spp., Heterakis spp.
Aus der Ordnung der Ascaridia z.B.: Ascaris spp., Toxascaris spp., Toxocara spp., Parascaris spp., Anisakis spp., Ascaridia spp.
Aus der Ordnung der Spirurida z.B.: Gnathostoma spp., Physaloptera spp., Thelazia spp., Gongylonema spp., Habronema spp., Parabronema spp., Draschia spp., Dracunculus spp.
Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.
Aus der Ordnung der Gigantorhynchida z.B.: Filicollis spp., Moniliformis spp., Macracanthorhynchus spp., Prosthenorchis spp.
Bei Einsatz geeigneter Wirkstoffkombinationen kann man das gesamte Spektrum an oben aufgeführten Endoparasiten abdecken.
Zu den Nutz- und Zuchttieren gehören Säugetiere wie z.B. Rinder, Pferde, Scbafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße.
Zu Labor- und Versuchstieren gehören Mäuse, Ratten, Meerschweinchen, Goldhamster, Hunde und Katzen.
Zu den Hobbytieren gehören Hunde und Katzen.

Die Anwendung kann sowohl prophylaktisch als auch therapeutisch erfolgen. Ganz besonders bevorzugt ist die Anwendung am Hund.

Pyrrolidon-Lösungsmittel sind pharmazeutisch verträgliche Lösungsmittel, die sich vom Pyrrolidon ableiten. Vorzugsweise handelt es sich um ein Pyrrolidon, das ein oder mehrere Alkylsusbtituenten mit bis zu 4 Kohlenstoffatomen tragen kann. Besonders bevorzugt ist das Pyrrolidon-Lösungsmittel ein 2-Pyrrolidon, das gegebenenfalls am Ringstickstoff einen Alkylsubstituenten mit 1 bis 4 Kohlenstoffatomen und gegebenenfalls an einer weiteren Ringposition einen weiteren Alkylsubstituenten mit 1 bis 4 Kohlenstoffatomen trägt. Beispiele sind 1,5-Diethyl-2-pyrrolidon, N-Ethyl-2-pyrrolidon. Insbesondere bevorzugt sind 2-Pyrrolidone, die gegebenenfalls nur einen Alkylsubstituenten am Stickstoff tragen, beispielsweise 2-Pyrrolidon und insbesondere N-Methylpyrrolidon.

Die erfindungsgemäßen Mittel enthalten das Pyrrolidon-Lösungsmittel oder Gemische davon üblicherweise in Mengen von 99 Gew:-% bis 20 Gew.-%, bevorzugt 96 Gew.-% bis 35 Gew.-%, besonders bevorzugt 90 Gew.-% bis 65 Gew.-% .

Insbesondere bei Mitteln, die cyclische Depsipeptide enthalten ist der Einsatz eines üblichen Antioxidans wie z. B. BHA, BHT oder Propylgallat vorteilhaft.

Die erfindungsgemäßen Mittel können zusätzlich Synergisten oder weitere Wirkstoffe, z.B. solche die gegen pathogene Endoparasiten wirken, enthalten. Solche Wirkstoffe sind z.B. L-2,3,5,6-Tetrahydro-6-phenyl-imidazothiazol, Benzimidazolcarbamate wie Febantel, ferner Pyrantel, Epsiprantel oder makrocyclische Lactone wie z. B. Avermectin, Ivermectin oder Selamectin.

Anwendungsfertige Zubereitungen enthalten die Wirkstoffe im Allgemeinen in Konzentrationen von jeweils 0,01 - 25 Gew.%, bevorzugt von 0,1-20 Gewichtsprozent.

Die cyclischen Depsipeptide werden üblicherweise in Mengen von 0,1 bis 8 Gew.-%, bevorzugt 1 bis 6 Gew.-% eingesetzt.

Praziquantel wird üblicherweise in Mengen von 1 bis 25 Gew.-%, bevorzugt 5 bis 15 Gew.-%, besonders bevorzugt 6 bis 14 Gew.-% eingesetzt.

Die Mittel werden durch Mischen der Komponenten in den entsprechenden Mengen in geeigneten Geräten hergestellt. Vorzugsweise geht man dabei so vor, dass man die flüssigen Komponenten mischt und dann die festen Komponenten zugibt und eine homogene Lösung herstellt.

Im allgemeinen hat es sich als vorteilhaft erwiesen Mengen der erfindungsgemäßen Mischung von etwa 1 bis etwa 100 mg Wirkstoff je kg Körpergewicht pro Tag zur Erzielung wirksamer Ergebnisse zu verabreichen. Bevorzugt sind 1 bis 10 mg Wirkstoff je kg Körpergewicht.

Die folgenden Beispiele erläutern die Erfindung.

### Beispiele

Zur Herstellung der folgenden Formulierungen wurden zunächst die flüssigen Bestandteile gemischt und dann unter Rühren die festen Bestandteile darin gelöst. Alle Prozentangaben sind Gewichtsprozent bezogen auf das Gesamtgewicht der fertigen Formulierung.

### Beispiel 1

| | |
|---|---|
| 4 % | PF 1022 |
| 8 % | Praziquantel |
| 79,1% | N-Methylpyrrolidon |
| 4,4 % | Limonen |
| 4,4 % | Ölsäure |
| 0,1% | BHA |

### Beispiel 2

| | |
|---|---|
| 4 % | Emodepside |
| 8 % | Praziquantel |
| 79,1 % | N-Methylpyxrolidon |
| 4,4 % | Limonen |
| 4,4 % | Ölsäure |
| 0,1 % | BHA |

### Beispiel 3

| | |
|---|---|
| 7% | Emodepside |
| 14 % | Praziquantel |
| 68,9 % | N-Methylpyrrolidon |
| 5% | Limonen |
| 5% | Ölsäure |
| 0,1% | BHA |

### Beispiel 4

| | |
|---|---|
| 4 % | Emodepside |
| 8 % | Praziquantel |
| 72,9 % | N-Methylpyrrolidon |
| 5 % | Limonen |
| 5 % | Ölsäure |
| 5.% | Isostearylalkohol |
| 0,1 % | BHA |

### Beispiel 5

| | |
|---|---|
| 4 % | Emodepside |
| 8 % | Praziquantel |
| 67,9 % | N-Methylpyrrolidon |
| 5% | Limonen |
| 5 % | Ölsäure |
| 10 % | Isostearylalkohol |
| 0,1% | BHA |

### Vergleichsbeispiel 6

| | |
|---|---|
| 4 % | Emodepside |
| 8 % | Praziquantel |
| 77,9 % | N-Methylpyrrolidon |
| 10 % | Isostearylalkohol |
| 0,1 % | BHA |

### Vergleichsbeispiel 7

| | |
|---|---|
| 4 % | Emodepside |
| 8 % | Praziquantel |
| 67,9% | N-Methylpyrrolidon |
| 20 % | Isostearylalkohol |
| 0,1 % | BHA |

### Vergleichsbeispiel 8

| | |
|---|---|
| 8 % | Praziquantel |
| 87 % | N-Methylpyrrolidon |
| 5% | Ölsäure |

### Beispiel 9

| | |
|---|---|
| 4% | Emodepside |
| 8 % | Praziquantel |
| 70,9 %. | N-Methylpyrrolidon |
| 5% | Limonen |
| 4,4% | Ölsäure |
| 6,5 % | Isostearylalkohol |
| 1,5% | Caprylsäure |
| 0,1 % | BHA |

### Beispiel 10

| | |
|---|---|
| 8 % | Praziquantel |
| 87 % | N-Methylpyrrolidon |
| 5% | Limonen |

### Beispiel 11

| | |
|---|---|
| 8 % | Praziquantel |
| 83% | N-Methylpyrrolidon |
| 4 % | Limonen |
| 4% | Ölsäure |

### Beispiel 12

| | |
|---|---|
| 4%. | Emodepside |
| 8 % | Praziquantel |
| 72,9 % | N-Methylpyrrolidon |
| *15%* | Limonen |
| 0,1% | BHA |

### Beispiel 13

| | |
|---|---|
| 8% | Praziquantel |
| 77% | N-Methylpyrrolidon |
| 15 % | Limonen |

### Beispiel 14

| | |
|---|---|
| 8% | Praziquantel |
| 72% | N-Methylpyrrolidon |
| 20 % | Limonen |

### Beispiel 15

| | |
|---|---|
| 4 % | Emodepside |
| 8 % | Praziquantel |
| 67,9 % | N-Methylpyrrolidon |
| 20 % | Limonen |
| 0,1 % | BHA |

### Beispiel 16

| | |
|---|---|
| 8 % | Praziquantel |
| 78% | N-Methylpyrrolidon |
| 4,4% | Limonen |
| 4,4% | Ölsäure |
| 5 % | Isostearylalkohol |

### Beispiel 17

| | |
|---|---|
| 4 % | Emodepside |
| 8 % | Praziquantel |
| 72,9 % | N-Methylpyrrolidon |
| 5% | Limonen |
| 5 % | Linolsäure |
| 5% | Isostearylalkohol |
| 0,1% | BHA |

### Beispiel 18

| | |
|---|---|
| 6% | Emodepside |
| 12% | Praziquantel |
| 71,9% | N-Methylpyrrolidon |
| 5% | Limonen |
| 5% | Ölsäure |
| 0,1% | BHA |

### Biologisches Beispiel

Die Lösungen aus Beispiel 2 oder 3 wurden auf das Rückenfell der mit Parasiten infizierten Tiere gegeben. Details können der folgenden Tabelle entnommen werden:

| **Formulierung** | **Tier** | **Parasit** | **Anzahl behandelter Tiere** | **Dosis (pro kg Körpergewicht)** | **Wirkung gegen Nematoden/ Cestoden** |
|---|---|---|---|---|---|
| Bsp. 2 | Hunde | *Ancylostoma caninum* | 6 | 12 mg Emodepsid 24 mg Praziquantel | 100 % |
| | | *U. stenocephala* | 6 | | 100 % |
| | | *T. vulpis* | 6 | | 90 % |
| | | *T. canis* | 6 | | 100 % |
| | | *Taenia sp.* | 6 | | 100 % |
| Bsp. 3 | Hunde | *D. caninum* | 6 | 24 mg Praziquantel 12 mg Emodepsid | 98 % |
| | | *T. vulpis* | 6 | | 100 % |

## Patentansprüche

1. Mittel enthaltend:
• als Wirkstoff ein cyclisches Depsipeptid und/oder Praziquantel
• ein Pyrrolidon-Lösungsmittel
• einen Terpen-Kohlenwasserstoff als Terpen-Penetrationsenhancer.

2. Mittel gemäß Anspruch 1, das als Terpen-Penetrationsenhancer einen Terpen-Kohlenwasserstoff mit einem Grundgerüst mit 10 Kohlenstoffatomen enthält.

3. Mittel gemäß Anspruch 2, das als Terpen-Penetrationsenhancer Limonen enthält.

4. Mittel gemäß einem der vorstehenden Ansprüche, enthaltend einen Terpen-Kohlenwasserstoff und eine als Penetrationsenhancer wirkende aliphatische Fettsäure.

5. Mittel gemäß Anspruch 4, worin die als Penetrationsenhancer wirkende aliphatische Fettsäure Linolsäure oder Ölsäure ist.

6. Mittel gemäß Anspruch 5, worin die als Penetrationsenhancer wirkende aliphatische Fettsäure Ölsäure ist.

7. Mittel gemäß einem der vorstehenden Ansprüche, enthaltend als Wirkstoff ein cyclisches Depsipeptid.

8. Mittel gemäß einem der vorstehenden Ansprüche, enthaltend als Wirkstoff Emodepside.

9. Mittel gemäß einem der Ansprüche 1 bis 9, enthaltend ein cyclisches Depsipeptid und Praziquantel.

10. Mittel gemäß Anspruch 8 oder 9, enthaltend Emodepside und Praziquantel.

11. Verfahren zur Herstellung der Mittel gemäß einem der Ansprüche 1 bis 10, **dadurch gekennzeichnet, dass** man die Wirkstoffe mit den Lösungsmitteln und gegebenenfalls weiteren Hilfsstoffen vermischt.

12. Verwendung von Mitteln gemäß einem der Ansprüche 1 bis 10 zur Herstellung eines Arzneimittels zur Bekämpfung von Endoparasiten.

## Claims

1. Composition, comprising:
• as active compound a cyclic depsipeptide and/or praziquantel
• a pyrrolidone solvent
• a terpene hydrocarbon as terpene penetration enhancer.

2. Composition according to Claim 1 which comprises, as terpene penetration enhancer, a terpene hydrocarbon having a skeleton of 10 carbon atoms.

3. Composition according to Claim 2 which comprises, as terpene penetration enhancer, limonene.

4. Composition according to any of the preceding claims, comprising a terpene hydrocarbon and an aliphatic fatty acid acting as penetration enhancer.

5. Composition according to Claim 4 wherein the aliphatic fatty acid acting as penetration enhancer is linoleic acid or oleic acid.

6. Composition according to Claim 5 wherein the aliphatic fatty acid acting as penetration enhancer is oleic acid.

7. Composition according to any of the preceding claims, comprising, as active compound, a cyclic depsipeptide.

8. Composition according to any of the preceding claims, comprising, as active compound, emodepside.

9. Composition according to any of Claims 1 to 9, comprising a cyclic depsipeptide and praziquantel.

10. Composition according to Claim 8 or 9, comprising emodepside and praziquantel.

11. Process for preparing the compositions according to any of Claims 1 to 10, **characterized in that** the active compounds are mixed with the solvents and, if appropriate, further auxiliaries.

12. Use of compositions according to any of Claims 1 to 10 for preparing a medicament for controlling endoparasites.

## Revendications

1. Composition contenant :
• comme substance active, un depsipeptide et/ou du praziquantel
• un solvant consistant en une pyrrolidone
• un hydrocarbure terpénique comme agent terpénique facilitant la pénétration

2. Composition suivant la revendication 1, qui contient comme agent terpénique facilitant la pénétration un hydrocarbure terpénique dont le squelette de base comprend 10 atomes de carbone.

3. Composition suivant la revendication 2, qui contient du limonène comme agent terpénique facilitant la pénétration.

4. Composition suivant l'une des revendications précédentes, contenant un hydrocarbure terpénique et un acide gras aliphatique agissant comme agent facilitant la pénétration.

5. Composition suivant la revendication 4, dans laquelle l'acide gras aliphatique agissant comme agent facilitant la pénétration est l'acide linoléique ou l'acide oléique.

6. Composition suivant la revendication 5, dans laquelle l'acide gras aliphatique agissant comme agent facilitant la pénétration est l'acide oléique.

7. Composition suivant l'une des revendications précédentes, contenant comme substance active un depsipeptide cyclique.

8. Composition suivant l'une des revendications précédentes, contenant comme substance active de l'émodepside.

9. Composition suivant l'une des revendications 1 à 9, contenant un depsipeptide cyclique et du praziquantel.

10. Composition suivant la revendication 8 ou 9, contenant de l'émodepside et du praziquantel.

11. Procédé de préparation des compositions suivant l'une des revendications 1 à 10, **caractérisé en ce qu'**on mélange les substances actives avec les solvants et, le cas échéant, avec d'autres substances actives.

12. Utilisation de compositions suivant l'une des revendications 1 à 10 pour la préparation d'un médicament destiné à combattre des endoparasites.
